# EUROPÄISCHE PATENTANMELDUNG

(11) **EP 0 789 034 A2**
(43) Veröffentlichungstag der Anmeldung: **13.08.1997**
(21) Anmeldenummer: 97101242.2
(22) Anmeldetag: 28.01.1997
(51) Int. Cl.: C08B 37/08, A61K 7/48, A61K 31/73

(54) **Biopolymere mit verbesserter Tensidlöslichkeit**

(30) Priorität: 06.02.1996 DE 19604180
(71) Anmelder: Henkel Kommanditgesellschaft auf Aktien, 40589 Düsseldorf-Holthausen (DE)
(72) Erfinder: Möller, Hinrich, Dr., 40789 Monheim (DE); Wachter, Rolf, Dr., 40595 Düsseldorf (DE)

(57) **Zusammenfassung**

Es werden neue Biopolymere mit verbesserter Löslichkeit in anionischen und nichtionischen Tensiden vorgeschlagen, die man erhält, indem man kationische Biopolymere vom Chitosantyp mit Dicarbonsäureanhydriden derivatisiert.

## Beschreibung

### Gebiet der Erfindung

Die Erfindung betrifft neue Biopolymere mit verbesserter Löslichkeit in Tensiden, die man erhält, indem man kationische Biopolymere vom Chitosantyp mit Dicarbonsäureanhydriden derivatisiert, ein Verfahren zur Herstellung der Derivate sowie deren Verwendung zur Herstellung von kosmetischen und/oder pharmazeutischen Zubereitungen.

### Stand der Technik

Bei der Herstellung moderner Hautpflegemittel werden vielfach Stoffe mitverwendet, deren Aufgabe es ist, den Feuchtigkeitsverlust der Haut zu vermindern bzw. zu regulieren. Typische Beispiele für kosmetische Zusatzstoffe dieser Art sind Hyaluronsäure und insbesondere Chitosan, ein kationisches Biopolymer, das aus maritimem Chitin gewonnen wird. Neben den Feuchtigkeitsregulatoren können die Formulierungen weitere Inhaltsstoffe und in aller Regel anionische und/oder nichtionische Tenside bzw. Emulgatoren enthalten. In diesen Fällen kann es zu einer Wechselwirkung zwischen Tensiden und Chitosanen kommen, was zu einer unerwünschten Trübung der ansonsten klaren Formulierung führt und die Vermarktung der Produkte erschwert.

Die Aufgabe der Erfindung hat somit darin bestanden, Chitosane in einer solchen Weise zu derivatisieren, daß sie in Tensidlösungen auch unter alkalischen Bedingungen klar löslich sind, ohne daß sich das Eigenschaftsbild der Polymere dabei nachteilig verändert.

### Beschreibung der Erfindung

Gegenstand der Erfindung sind Biopolymere mit verbesserter Tensidlöslichkeit, dadurch erhältlich, daß man Chitosane mit cyclischen Dicarbonsäureanhydriden umsetzt.

Überraschenderweise wurde gefunden, daß die Anlagerung von cyclischen Dicarbonsäureanhydriden an die kationischen Biopolymere deren Klarlöslichkeit in anionischen und nichtionischen Tensiden auch unter alkalischen Bedingungen ermöglicht, ohne daß dadurch die anwendungstechnischen Eigenschaften der Polymeren nachteilig beeinflußt werden. Insbesondere bei Einsatz von Biopolymeren mit niedrigem Molekulargewicht werden anionische Derivate erhalten, die ein besonders vorteilhaftes Löslichkeitsverhalten zeigen und von denen sich außerdem problemlos höherkonzentrierte Lösungen herstellen lassen.

Ein weiterer Gegenstand der Erfindung betrifft ein Verfahren zur Herstellung von Biopolymeren mit verbesserter Tensidlöslichkeit, bei dem man Chitosane mit cyclischen Dicarbonsäureanhydriden umsetzt.

### Chitosane

Kationische Biopolymere vom Typ der Chitosane werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

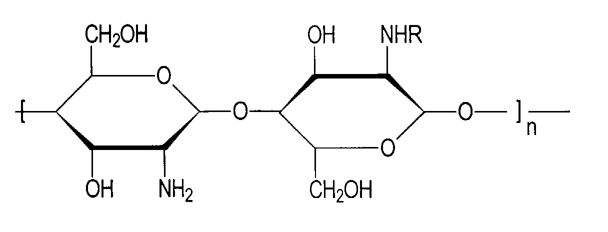

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag** **Chemie, 1986, S. 231-332)**. Übersichten zu diesem Thema sind auch beispielsweise von B.Gesslein et al. in **HAPPI 27, 57 (1990)**, O.Skaugrud in **Drug Cosm.Ind. 148, 24 (1991)** und E.Onsoyen et al. in **Seifen-Öle-Fette-Wachse 117, 633 (1991)** erschienen.

Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR-A 27 01 266** bekannt. Vorzugsweise werden Chitosane eingesetzt, die ein durchschnittliches Molekulargewicht im Bereich von 10.000 bis 1.200.000 und insbesondere von 800.000 bis 1.000.000 Dalton sowie einen Deacetylierungsgrad im Bereich von 75 bis 95 und insbesondere 82 bis 88 % aufweisen. Neben diese hochmolekularen Biopolymeren können auch niedermolekulare Spezies mit einem durchschnittlichen Molekulargewicht von 10.000 bis 100.000 und vorzugsweise 30.000 bis 60.000 bei gleichem Deacetylierungsgrad eingesetzt werden, die man durch Abbau der hochmolekularen Homologen mit Wasserstoffperoxid erhält.

### Cyclische Dicarbonsäureanhydride

Die cyclischen Dicarbonsäureanhydride, die im Sinne der Erfindung für die Derivatisierung in Betracht kommen, leiten sich vorzugsweise von linearen oder verzweigten, gesättigten oder ungesättigten, aliphatischen oder aromatischen Dicarbonsäuren mit 2 bis 12 Kohlenstoffatomen ab. Typische Beispiele sind Anhydride auf Basis von Maleinsäure, Bernsteinsäure, Citraconsäure, Itaconsäure, Glutarsäure, Methylbernsteinsäure, Phthalsäure, 1,2-Cyclopentandicarbonsäure und 1,2-Cyclohexandicarbonsäure. Bevorzugt ist der Einsatz von Malein- und Bernsteinsäureanhydrid sowie deren Mischungen.

### Verfahren

Für die Umsetzung werden die Chitosane vorzugsweise in 0,1 bis 5 und insbesondere 0,5 bis 3 Gew.-%iger Lösung vorgelegt, wobei sich als Lösungsmittel beispielsweise wäßrige Salzsäure, Essigsäure oder Glycolsäure bewährt haben. Das molare Einsatzverhältnis der Chitosane - bezogen auf den Monomerbaustein - und der Dicarbonsäureanhydride kann 1 : 0,05 bis 1 : 1,5 und insbesondere 1 : 0,1 bis 1 : 0,5 betragen. Überraschenderweise zeigt such, daß auch stark unterstöchiometrische Umsetzungen mit cyclischen Anhydriden zu Derivaten führen, die ganz ähnliche Löslichkeits- und Kompatibilitätsverbesserungen aufweisen, wie etwa 1:1-Umsetzungsprodukte. Gerade diese geringgradig carboxyacylierten Derivate zeichnen sich dadurch aus, daß sie einerseits noch eine große Zahl kationischer Zentren besitzen und andererseits für Folgereaktionen in einem breiten pH-Wertbereich zur Verfügung stehen.

Vorzugsweise wird die Reaktion bei einem pH-Wert im Bereich von 2,0 bis 6,5 und vorzugsweise 3,0 und 5,5 durchgeführt. Dazu empfiehlt es sich, bei dem pH-Wert zu arbeiten, der sich beim Auflösen des Biopolymers mit etwa einem Säureäquivalent pro Aminogruppe einstellt und bei etwa 3 bis 4 liegt. Zum Lösen der Polymere können wäßrige Mineralsäuren oder organische Säuren eingesetzt werden wie beispielsweise Salzsäure, Glycolsäure oder die Säure des für die nachfolgende Umsetzung eingesetzten Anhydrids, wobei die Konzentration des Polymeres 0,5 bis 40 und vorzugsweise 3 bis 20 Gew.-% betragen kann.

Falls erforderlich, kann der pH-Wert durch Zugabe von wäßrigen Alkali- und/oder Erdalkalihydroxiden, -carbonaten und/oder -hydrogencarbonaten eingestellt werden. Die cyclischen Anhydride können in Lösungen in einem wassermischbaren Lösungsmittel wie beispielsweise Aceton, Tetrahydrofuran, 1,2-Dimethoxyethan, 2-Butanon, Ethylendiglycoldimethylether, N-Methylpyrrolidon oder dergleichen eingesetzt werden; es ist jedoch bevorzugt, sie in Substanz einzusetzen. Die Reaktion kann bei Temperaturen im Bereich von 0 bis 100 durchgeführt werden. Bevorzugt ist jedoch ein Bereich von 20 bis 50°C und eine Reaktionszeit von 0,5 bis 3 h. Wird ein Lösungsmittel mitverwendet, empfiehlt es sich, dieses nach Abschluß der Reaktion wieder im Vakuum abzutrennen.

### Gewerbliche Anwendbarkeit

Die neuen Biopolymerderivate verfügen über ausgezeichnete feuchtigkeitsregulierende und filmbildende Eigenschaften und eignen sich daher zur Herstellung von kosmetischen und/oder pharmazeutischen Zubereitungen, speziell Haut- und Haarpflegemitteln, in denen sie in Mengen von 0,1 bis 5 und vorzugsweise 0,5 bis 2 Gew.-% - bezogen auf die Mittel - enthalten sein können.

Die Zubereitungen können mit den anderen Inhaltsstoffen kompatible **Tenside** enthalten. Typische Beispiele sind Fettalkoholpolyglycolethersulfate, Monoglyceridsulfate, Mono- und/ oder Dialkylsulfosuccinate, Fettsäureisethionate, Fettsäuresarcosinate, Fettsäuretauride, Ethercarbonsäuren, Alkyloligoglucoside, Fettsäureglucamide, Alkylamidobetaine und/oder pflanzliche Proteinhydrolyse bzw. Proteinfettsäurekondensate.

Als weitere Hilfs- und Zusatzstoffe kommen Ölkörper, Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe in Betracht.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(b1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574** sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkyl-aminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acyliene Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

### Beispiel 1

Zu einer gerührten klaren Lösung von 10 g (46, 2 mmol) Chitosan (Hydagen® CMF, Molgewicht ca. 1.000.000 Dalton, Deacetylierungsgrad = 82 %) in 600 ml Wasser und 23,1 ml 2n Salzsäure wurden bei 30°C 4,65 g Natriumhydrogencarbonat in 400 ml Wasser gegeben, wobei sich ein pH-Wert von 6,1 einstellte und die Lösung austrübte. In die gerührte Mischung wurden 5,1 g (50,8 mmol) Bernsteinsäureanhydrid in 20 ml Aceton langsam zugetropft, wobei der pH-Wert in einem Bereich von 6,1 bis 6,4 gehalten wurde. Die allmählich klar werdende Mischung wurde noch 1,5 h bei 30°C gerührt und das Lösungsmittel dann im Vakuum entfernt. Es wurde eine gelig viskose, klare Masse erhalten.

### Beispiel 2

Beispiel 1 wurde wiederholt, die Menge an Bernsteinsäureanhydrid jedoch halbiert. Es wurde ein schwach trübes, viskoses, gelartiges Produkt erhalten, das bei pH-Werten oberhalb von 7 klar wurde.

### Beispiel 3

Beispiel 1 wurde wiederholt, jedoch anstelle des Bernsteinsäureanhydrids 51 mmol Maleinsäureanhydrid eingesetzt. Es wurde eine gelig viskose, klare Masse erhalten.

### Beispiel 4

Beispiel 1 wurde wiederholt, jedoch anstelle des Bernsteinsäureanhydrids 51 mmol Phthalsäureanhydrid eingesetzt. Es wurde eine gelig viskose, klare Masse erhalten.

### Beispiel 5

Zu der gerührten, klaren und viskosen Lösung von 105 g (548 mmol) abgebautem Chitosan (Hydagen® HCMF, Henkel KGaA, mittleres Molekulargewicht 50.000 Dalton, Deacetylierungsgrad 82 %) in 700 ml Wasser und 32,4 g (275 mmol) Bernsteinsäure wurden zwischen 25 und 30°C 54,8 g (548 mmol) gepulvertes Bernsteinsäureanhydrid portionsweise über einen Zeitraum von 2 h gegeben. Nach 20stündigem Rühren bei Raumtemperatur wurde bei einem pH-Wert von 3,6 eine klare und viskose Lösung erhalten. Die leicht gelbliche Farbe der erhaltenen Lösung, die beim Vermischen mit 25 Gew.-%iger Ethersulfat- oder 20 Gew.-% Dodecylglucosidlösung keine Trübung ergab und auch im alkalischen Milieu löslich war, glich der der Ausgangslösung.

### Beispiel 6

Beispiel 5 wurde wiederholt, jedoch 138 mmol Bernsteinsäureanhydrid eingesetzt. Es wurde eine klare Lösung erhalten, deren Eigenschaften gegenüber alkalischen pH-Werten und Aniontensiden mit denen des Präparats aus Beispiel 5 vergleichbar waren.

### Beispiele 7 bis 10, Vergleichsbeispiel V1

Jeweils 0,5 g der nach den Herstellbeispielen 1 bis 4 erhaltenen Biopolymeren (Beispiele 6 bis 9) sowie 0,5 g eines handelsüblichen Chitosans (Vergleichsbeispiel V1) wurden in 100 ml einer 25 Gew.-%igen Lösung von Kokosfettalkohol+2EO-sulfat-Natriumsalz (Texapon® NSO, Henkel KGaA) bzw. einer 20 Gew.-%igen Lösung von Kokosalkylglucosid (Plantaren® 1200, Henkel KGaA) gelöst. Im Fall der erfindungsgemäßen Dicarbonsäureanhydridderivatisierten Biopolymere wurden klare Lösungen erhalten, die auch im alkalischen Bereich nicht austrübten. Im Fall des Handelsproduktes resultierten in beiden Fällen trübe Lösungen.

## Patentansprüche

1. Biopolymere mit verbesserter Tensidlöslichkeit, dadurch erhältlich, daß man Chitosane mit cyclischen Dicarbonsäureanhydriden umsetzt.

2. Verfahren zur Herstellung von Biopolymeren mit verbesserter Tensidlöslichkeit, bei dem man Chitosane mit cyclischen Dicarbonsäureanhydriden umsetzt.

3. Verfahren nach Anspruch 2, **dadurch gekennzeichnet**, daß man Chitosane einsetzt, die ein durchschnittliches Molekulargewicht im Bereich von 10.000 bis 1.200.000 Dalton und einen Deacetylierungsgrad im Bereich von 75 bis 95 % einsetzt.

4. Verfahren nach Anspruch 2, **dadurch gekennzeichnet**, daß man Chitosane einsetzt, die ein durchschnittliches Molekulargewicht von 30.000 bis 100.000 und einen Deacetylierungsgrad von 75 bis 95 % aufweisen.

5. Verfahren nach Anspruch 2, **dadurch gekennzeichnet**, daß man Chitosane einsetzt, die ein durchschnittliches Molekulargewicht von 800.000 bis 1.000.000 und einen Deacetylierungsgrad von 75 bis 95 Gew.-% aufweisen.

6. Verfahren nach den Ansprüchen 2 bis 5, **dadurch gekennzeichnet**, daß man cyclische Dicarbonsäureanhydride einsetzt, die sich von linearen oder verzweigten, gesättigten oder ungesättigten, aliphatischen oder aromatischen Dicarbonsäuren mit 2 bis 12 Kohlenstoffatomen ableiten.

7. Verfahren nach den Ansprüchen 2 bis 6, **dadurch gekennzeichnet**, daß man die Chitosane - bezogen auf den Monomerbaustein - und die Dicarbonsäureanhydride im molaren Verhältnis von 1: 0,05 bis 1 : 1,5 einsetzt.

8. Verfahren nach den Ansprüchen 2 bis 7, **dadurch gekennzeichnet**, daß man die Reaktion bei einem pH-Wert im Bereich von 2,0 bis 6,5 durchführt.

9. Verwendung der Biopolymere nach Anspruch 1 zur Herstellung von kosmetischen und/oder pharmazeutischen Zubereitungen.
